(19) Europäisches Patentamt
European Patent Office
Office européen des brevets

(11)  **EP 2 678 661 B1**

(12)  **FASCICULE DE BREVET EUROPEEN**

(45) Date de publication et mention
de la délivrance du brevet:
**22.04.2015 Bulletin 2015/17**

(21) Numéro de dépôt: **12706704.9**

(22) Date de dépôt: **16.02.2012**

(51) Int Cl.:
***G01N 21/03*** *(2006.01)*      ***G01N 21/64*** *(2006.01)*

(86) Numéro de dépôt international:
**PCT/IB2012/050721**

(87) Numéro de publication internationale:
**WO 2012/114237 (30.08.2012 Gazette 2012/35)**

(54) **PROCÉDÉ D'OBSERVATION D'UN ÉCHANTILLON**

VERFAHREN ZUR BEOBACHTUNG EINER PROBE

METHOD FOR OBSERVING A SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.02.2011 FR 1100522**

(43) Date de publication de la demande:
**01.01.2014 Bulletin 2014/01**

(73) Titulaires:
• **Commissariat à l'Énergie Atomique et aux Énergies Alternatives**
**75015 Paris (FR)**
• **Biomérieux**
**69280 Marcy L'Etoile (FR)**

(72) Inventeurs:
• **DUPOY, Mathieu**
**38100 Grenoble (FR)**
• **JOLY, Pierre**
**38100 Grenoble (FR)**
• **MALLARD, Frédéric**
**38340 Voreppe (FR)**
• **MARCOUX, Pierre**
**38120 SAINT EGREVE (FR)**
• **MATHEY, Raphaël**
**38100 Grenoble (FR)**

• **NOVELLI-ROUSSEAU, Armelle**
**38180 Seyssins (FR)**

(74) Mandataire: **Reboussin, Yohann Mickaël Noël et al**
**Cabinet Orès**
**36, rue de St Pétersbourg**
**75008 Paris (FR)**

(56) Documents cités:
**JP-A- 2008 249 446      US-A1- 2004 109 038**
**US-A1- 2005 221 339**

• **Boris Rotman: "Measurement of activity of single molecules of [beta]-D-Galactosidase", Proceedings of the Natl.Acad. Sci. USA, Vol.47, no.12, 1 décembre 1961 (1961-12-01), pages 1981-1991, XP055005802, Extrait de l'Internet: URL:http://www.ncbi.nlm.nih.gov/pmc/articl es/PMC223251/pdf/pnas00216-0111.pdf [extrait le 2011-08-30] cité dans la demande**
• **ASHLEIGH B. THEBERGE ET AL: "Microdroplets in Microfluidics: An Evolving Platform for Discoveries in Chemistry and Biology", ANGEWANDTE CHEMIE INTERNATIONAL EDITION, vol. 49, no. 34, 21 juin 2010 (2010-06-21) , pages N/A-N/A, XP055005795, ISSN: 1433-7851, DOI: 10.1002/anie.200906653 cité dans la demande**

**Description**

L'invention porte sur un procédé d'observation d'un échantillon, notamment pour des applications biologiques ou chimiques telles que la détection ou numération de microorganismes et l'étude de la cinétique de réactions chimiques.

Un procédé selon un mode préféré de réalisation est basé sur l'utilisation d'une microgouttelette pour contenir l'échantillon à observer, ou constituant elle-même ledit échantillon. L'article de A. B. Theberge et al. « Microdroplets in Microfluidics : An Evolving Platform for Discoveries in Chemistry and Biology », Angew. Chem. Int. Ed. 2010, 49, 5846 - 5868 présente une revue des techniques microfluidiques basées sur l'emploi de microgouttelettes liquides pour une variété d'applications à la chimie et en biologie. On entend par « microgouttelettes » des gouttelettes de diamètre (ou, plus généralement, de dimension principale) compris entre 1 $\mu$m et 100 $\mu$m.

Le document US2005/0221339 décrit un procédé de détection optique de composants chimiques dans des microgouttelettes manipulées dans un système microfluidique.

L'article de B. Rotman « Measurement of activity of single molecules of $\beta$-D-Galactosidase », Proceedings of the Natl. Acad. Sci. USA, 1961, 47 pp. 1981 - 1991 décrit un procédé de détection de molécules isolées d'un enzyme. Dans ce procédé, une solution aqueuse contenant l'enzyme (très diluée) et un substrat fluorogène de cette enzyme est nébulisée à l'intérieur d'un faible volume d'huile de silicone confiné entre deux lames de microscope. Etant donné que l'huile est moins dense que l'eau et immiscible avec elle, la solution forme des microgouttelettes qui se déposent sur la surface supérieure de la lame de microscope inférieure. Puis les gouttelettes sont analysées par microfluorométrie à l'aide d'un microscope. Les gouttelettes contenant au moins une molécule de l'enzyme apparaissent comme des disques lumineux, alors que les gouttelettes n'en contenant pas apparaissent sous la forme d'anneaux lumineux.

La principale limitation de ces procédés réside dans la faible intensité de la lumière émise par fluorescence. Des problèmes semblables se posent lorsqu'il s'agit d'observer la lumière diffusée (diffusion élastique ou Raman) par un objet de dimensions microscopiques contenu dans une gouttelette.

L'invention vise à surmonter cette limitation en proposant une configuration d'observation d'un échantillon améliorant l'efficacité de collection de la lumière émise ou diffusée par ce dernier. L'invention n'est d'ailleurs pas limitée à l'utilisation d'une gouttelette liquide, mais s'étend au cas où cette dernière est remplacée par un volume d'un milieu solide ou d'un gel.

Un objet de l'invention est donc un procédé d'observation d'un échantillon comprenant les étapes consistant à :

a) procurer un volume d'un milieu sensiblement transparent ayant un premier indice de réfraction $n_{int}$ et présentant une première surface convexe, arrondie ou à facettes, et une deuxième surface sensiblement plane, ladite première surface séparant ledit volume d'un milieu ambiant présentant un deuxième indice de réfraction $n_{ext}$, l'échantillon à observer étant constitué par ledit volume, ou disposé à son intérieur ou déposé sur ladite première surface ; et
b) observer ledit échantillon ;
caractérisé en ce que :

- lors de l'étape b), on observe ledit échantillon à travers ladite deuxième surface sensiblement plane ;

et en ce que :

- ledit deuxième indice de réfraction est inférieur audit premier indice.

Le procédé comprend en plus une étape c) de détermination de la présence d'une substance ou d'un objet dans l'échantillon en fonction du résultat de l'observation dudit échantillon lors de l'étape b). L'objet peut être notamment une espèce biologique, telle qu'un micro-organisme, par exemple une bactérie, une levure, un champignon, un virus, etc. Une substance à détecter peut être ; par exemple, solubilisée ou dispersée dans un milieu transparent ou translucide.

L'échantillon peut être un liquide, par exemple un liquide biologique, un liquide ionique, une solution aqueuse, ou bien un gel. Dans le cas d'un gel, la viscosité peut par exemple être comprise entre 1 cP (centpoise) et 1000 cP, c'est-à-dire entre $10^{-3}$ Pa·s et 1 Pa·s.

Comme cela sera expliqué plus en détail par la suite, le fait que l'indice de réfraction du milieu ambiant soit inférieur à celui du volume assure un guidage partiel de la lumière émise ou diffusée par l'échantillon le long de ladite première surface convexe (« bol de lumière »), ce qui améliore l'efficacité de sa détection du côté de la deuxième surface plane. Par contre, dans l'art antérieur (voir notamment l'article précité de B. Rotman) on utilise le plus souvent des « émulsions inversées » de solutions aqueuses dans une huile de plus fort indice optique, et/ou une observation de l'échantillon par la face convexe.

Le volume peut présenter, typiquement, un diamètre ou dimension principale comprise entre 1 $\mu$m et 1 mm et de préférence entre 10 et 500 $\mu$m.

Selon différents modes de réalisation de l'invention :

- Ledit volume peut présenter une forme sensiblement hémisphérique. En effet, il s'agit là de la géométrie qui maximise la collection de la lumière émise ou diffusée par l'échantillon.
- Ledit volume peut être une gouttelette adhérée à un substrat transparent formant ladite deuxième surface sensiblement plane.
- Ladite étape a) peut comporter la réalisation d'une pluralité de gouttelettes formant desdits volumes dont au moins certains contiennent au moins un objet ou une substance à détecter ; et ladite étape b) peut comporter l'observation simultanée d'une pluralité desdites gouttelettes.
- Ledit volume peut, au contraire, être un volume solide, auquel cas l'échantillon peut être constitué par une couche déposée sur sa première surface. Avantageusement, ladite couche peut être une couche fonctionnalisée, sensible à un analyte pouvant être contenu dans ledit milieu ambiant. Lorsque le volume est solide, l'échantillon peut aussi être un échantillon liquide ou visqueux placé au contact de la première surface du volume.
- Ledit volume peut être en un matériau sensible à un analyte pouvant être contenu dans ledit milieu ambiant
- Ledit milieu ambiant peut être l'air.
- Ladite étape b) peut comporter une opération d'éclairage dudit volume. De préférence, ledit éclairage peut être effectué à travers ladite deuxième surface sensiblement plane, de manière à bénéficier à son tour de l'effet de guidage de la lumière mentionné plus haut. Selon différentes variantes :
- Ladite étape b) peut comporter la détection d'un signal optique, par exemple de fluorescence. Dans ce cas, ladite étape b) peut être mise en oeuvre par microscopie à épifluorescence.
- Ladite étape b) peut comporter la détection d'un signal de diffusion Raman.
- Ladite étape b) peut être mise en oeuvre par microscopie à réflexion ou à transmission.

D'autres caractéristiques, détails et avantages de l'invention ressortiront à la lecture de la description faite en référence aux dessins annexés donnés à titre d'exemple et qui représentent, respectivement :

- La figure 1, le cheminement de différents rayons de lumière se propageant à l'interface entre une gouttelette et un milieu ambiant de plus faible indice de réfraction, tel que l'air ;
- Les figures 2A et 2B, un tracé de rayons issus d'un point de la gouttelette situé à proximité de ladite interface ;
- La figure 3, un tracé de rayons montrant le confinement de la lumière entrant dans une telle gouttelette à proximité de sa périphérie dans le plan passant par la source lumineuse et perpendiculaire à ladite deuxième surface.
- Les figures 4A et 4B, deux tracés de rayons pour la configuration d'observation décrite dans l'article précité de B. Rotman ;
- La figure 5, un essai comparatif illustrant les avantages apportés par l'invention ;
- La figure 6, un montage expérimental pour la mise en oeuvre d'un procédé selon l'invention ;
- Les figures 7A et 7B, respectivement, le schéma d'un procédé selon un premier mode de réalisation de l'invention et une image acquise grâce à ce procédé ;
- Les figures 8A et 8B, respectivement, le schéma d'un procédé selon un deuxième mode de réalisation de l'invention et une image acquise grâce à ce procédé ;
- Les figures 9A et 9B, respectivement, le schéma d'un procédé selon un troisième mode de réalisation de l'invention, une image acquise grâce à ce procédé et un détail d'une telle image ;
- La figure 10, le schéma d'un procédé selon un quatrième mode de réalisation de l'invention ;
- La figure 11, le schéma d'un procédé selon un cinquième mode de réalisation de l'invention ; et
- Les figures 12A à 12D illustrent les conséquences des déviations de la forme du volume par rapport à une demi-sphère.

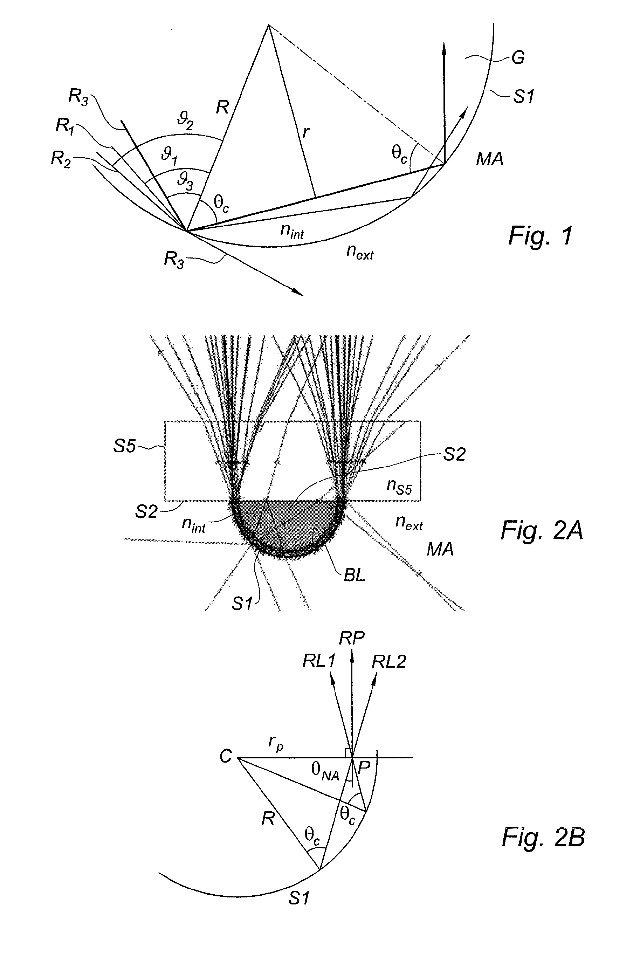

La figure 1 illustre le phénomène physique à la base de l'invention. On considère une gouttelette G d'un liquide présentant un indice de réfraction (réel) $n_{int}$, immergé dans un milieu ambiant MA, d'indice de réfraction (également réel) $n_{ext} < n_{int}$. Dans le cas d'une gouttelette d'eau dans l'air, on a $n_{int} = 1,33 < n_{ext} = 1$.

Sous l'effet de sa tension superficielle, la gouttelette prend une forme sensiblement sphérique ou en sphère tronquée, de diamètre R ; si R est de l'ordre de quelques micromètres, les déformations induites par les forces de pesanteur peuvent en effet être négligées.

Sur la figure 1 ont été représentés trois rayons de lumière, R1, R2 et R3, qui se propagent à l'intérieur de la gouttelette G, et qui rencontrent sa surface S1 avec un angle d'incidence $\theta_1$, $\theta_2$ et $\theta_3$ respectivement. D'une manière conventionnelle, l'angle d'incidence est défini comme étant l'angle formé entre le rayon et la normale à la surface.

Tous les rayons qui rencontrent la surface S1 avec un angle d'incidence supérieur à l'angle critique $\theta_c$, donné

par $\sin(\theta_c) = \dfrac{n_{ext}}{n_{int}}$, subissent une réflexion totale et restent piégés à l'intérieur de la gouttelette. Dans l'exemple de la figure 1, c'est le sort des rayons R1 (pour lequel $\theta_1 = \theta_c$) et R2 (pour lequel $\theta_2 > \theta_c$), tandis que le rayon R3 sort de la

gouttelette car $\theta_3 < \theta_c$.

Un simple raisonnement géométrique permet de montrer que les rayons subissant la réflexion totale restent confinés à proximité de la surface S1 de la gouttelette, et ne peuvent pas s'approcher de son centre à une distance inférieure à $r = R \dfrac{n_{ext}}{n_{int}}$ .

Pour comprendre en quoi cet effet peut aider l'observation d'objets tels que microorganismes ou de molécules fluorescentes, on considère la situation illustrée sur la figure 2, montrant une gouttelette G hémisphérique, adhérée à un support SS transparent et immergée dans un milieu MA tel que l'air. Comme dans le cas précédent $n_{ext} < n_{int}$; l'indice de réfraction $n_{ss}$ du substrat peut être inférieur ou supérieur à $n_{int}$; de préférence il ne sera pas trop élevé (par exemple, inférieur ou égal à $2n_{int}$) pour ne pas piéger à son tour la lumière. Des rayons lumineux sont émis par une molécule fluorescente située en un point proche de la surface hémisphérique S1 de la goutte (on indique par S2 la surface de contact avec le substrat). On peut voir qu'une partie significative de ces rayons sont guidés par l'interface air-eau dans une couche prenant la forme d'un « bol lumineux » BL. Ces rayons sortent de la goutte, au niveau de la surface plane S2, dans une zone de forme annulaire (qui peut aussi être appelée couronne circulaire), comprise entre un rayon interne $r = R \dfrac{n_{ext}}{n_{int}}$ et un rayon externe R. On comprend que cela améliore l'efficacité de collecte de la lumière. L'épaisseur du bol lumineux BL, que l'on peut également désigner par le terme enveloppe sphérique, est égale à R - r.

Pour préciser l'étendue angulaire du faisceau qui chemine dans le bol lumineux, la figure 2B s'intéresse aux rayons passant par un point fixe P, situé à la distance $r_P$ du centre C. Si $r_P$ vérifie la condition $r < r_P < R$, alors on peut montrer qu'il existe deux rayons RL1 et RL2 qui interceptent le cercle S1 avec l'angle d'incidence critique $\theta_c$ et que la bissectrice de RL1 et RL2, notée RP est orthogonale à CP. L'angle $\theta_{NA}$, entre RP et RL1 ou RL2, est tel que

$$\frac{\sin \theta_c}{r_P} = \frac{\sin(90° - \theta_{NA})}{R} \text{ , soit } \theta_{NA} = \cos^{-1}\left( \frac{n_{ext}}{n_{int}} \times \frac{R}{r_P} \right).$$ En termes d'optique géométrique, cela indique qu'à l'intérieur du volume G, le faisceau formé par les rayons passant par P a pour rayon principal RP (perpendiculaire à CP) et angle d'ouverture $\theta_{NA}$. Les valeurs limites de $\theta_{NA}$ sont : 0 (pour $r_P = r$) et $\theta_{NAMax} = 90° - \theta_c$ (pour $r_P = R$). C'est donc au bord de l'anneau que le faisceau a la plus grande ouverture numérique.

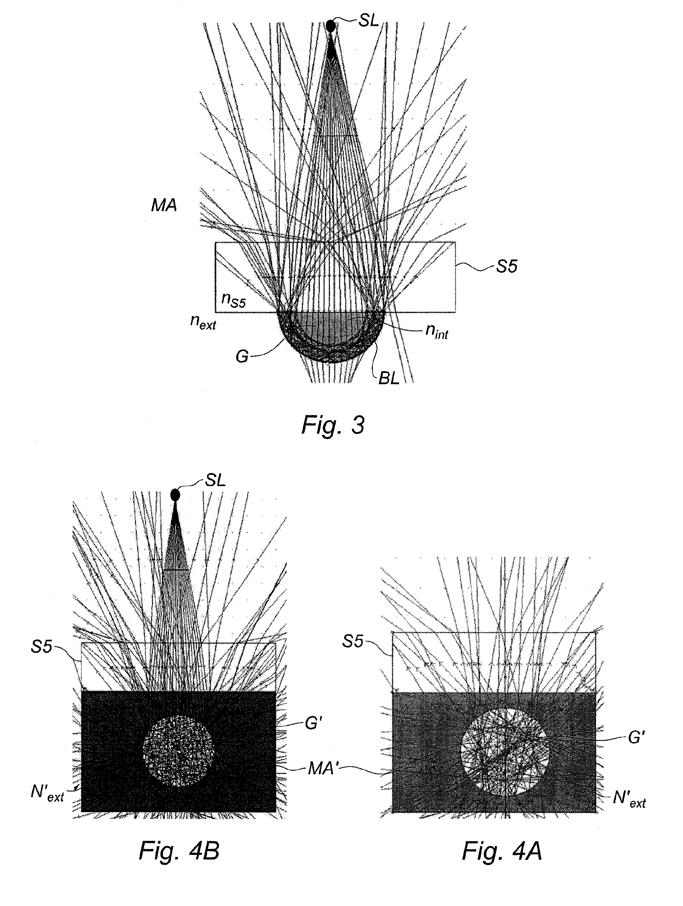

La figure 3 illustre la situation réciproque, dans laquelle une source lumineuse SL éclaire la gouttelette G à travers le substrat SS. De préférence, l'éclairage est tel qu'aucun cache n'est disposé entre la source de lumière SL et le substrat transparent SS. Aussi, le substrat SS et la gouttelette G ne sont pas éclairés selon une configuration dite de fond noir. Sur cette figure, les rayons qui pénètrent dans la gouttelette par la zone annulaire précitée de la surface plane S2 restent piégés dans le « bol lumineux » BL. Un échantillon, ou une portion d'un échantillon, situé à l'intérieur de ce « bol » est éclairé de manière beaucoup plus efficace que si ce piégeage de la lumière n'avait pas lieu.

Cet effet de confinement des rayons lumineux est dépendant de la condition $n_{ext} < n_{int}$, comme le montrent les figures 4A et 4B qui se rapportent à la situation opposée dans laquelle $n'_{ext} > n'_{int}$ (cas d'une gouttelette G' d'eau - $n_{int}' \approx 1,33$ - dans un bain MA' d'huile silicone - $n'_{ext} \approx 1,44$). On peut voir sur ces figures que dans ce cas, ni les rayons émis par une source extérieure SL, ni ceux issus d'un point de la surface de la gouttelette, ne restent piégés.

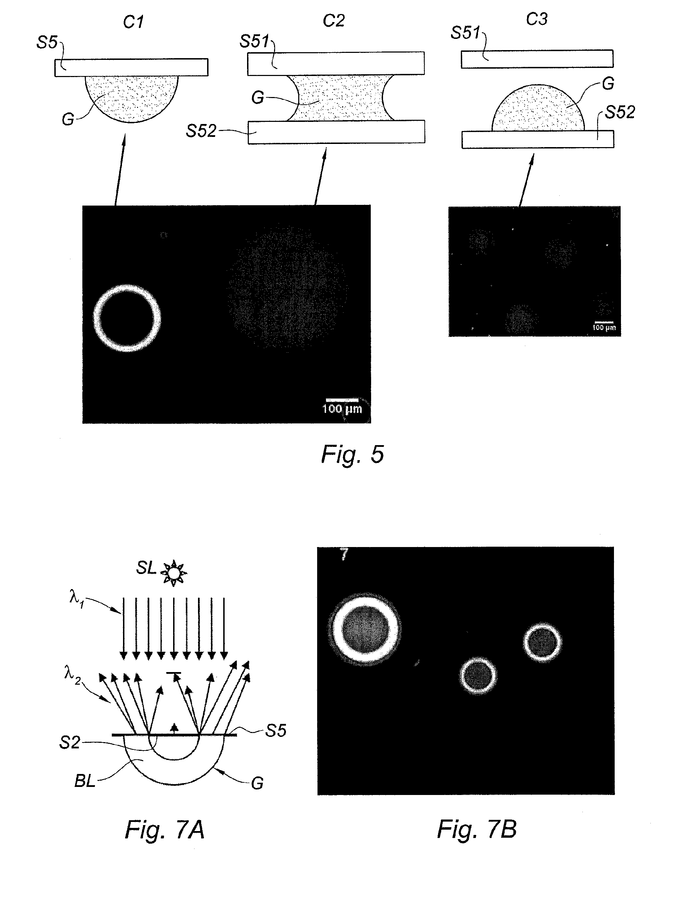

La figure 5 permet de comparer la configuration d'observation de l'invention (C1) à deux autres configurations : une gouttelette écrasée entre une lame - inférieure - et une lamelle - supérieure - de microscope (C2) et une gouttelette déposée sur une lame et observée du côté de sa surface convexe (C3). Dans tous les cas, la gouttelette est constituée d'une solution aqueuse d'un fluorophore 4MU (4-Methylumbelliferone) à 5μM. Les gouttelettes sont observées en microscopie par épifluorescence, c'est à dire avec excitation et détection du côté supérieur. On peut voir que la configuration C1 de l'invention permet de visualiser des anneaux très lumineux, tandis que dans les autres configurations la gouttelette fluorescente apparaît comme un disque très peu lumineux, qui se détache du fond avec difficulté. Des mesures expérimentales ont permis de montrer que la configuration de l'invention permet d'augmenter la quantité de lumière collectée d'un facteur 20 par rapport à la configuration « eau dans huile » utilisée par Rotman. Par quantité de lumière, on entend la quantité de lumière mesurée sur l'ensemble de la goutte, comprenant à la fois l'anneau lumineux et la zone centrale, beaucoup plus sombre. Par contre, si on ne prend en compte que la lumière émise dans un rayon circulaire de rayon compris entre $r = R \dfrac{n_{ext}}{n_{int}}$ et R, le gain atteint même un facteur 70.

Le cas d'une gouttelette hémisphérique est optimal, mais l'effet de confinement persiste même si la surface S1 s'écarte de cette forme nominale. Pour chiffrer les écarts tolérables, on distingue d'une part le fait que la surface S1 ne soit pas exactement une portion de sphère et d'autre part le fait que le volume G, globalement sphérique, ne soit pas tronqué exactement en milieu ou par une surface pas exactement plane.

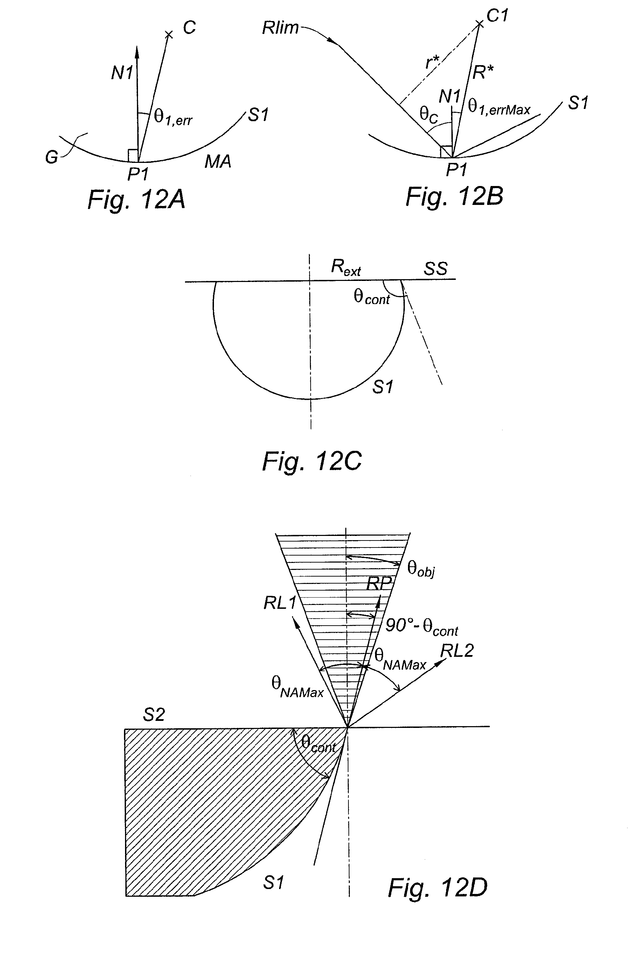

Comme illustré par la Figure 12A, on définit en chaque point P1 de S1, la normale N1, orientée vers l'intérieur de la goutte (de MA vers G). L'écart angulaire par rapport à une sphère de centre C est défini de la manière suivante : $\theta_{1,err}(P1,C)$, angle entre N1 et la droite (P1,C). Le centre des surfaces sphériques qui s'ajustent le mieux à S1 est le point C1 qui minimise $\iint_{P1 \in S1} \theta_{1,err}(P1, C1) dS1$ (les angles ne sont pas orientés, donc leurs valeurs sont toujours positives) Une fois ce point trouvé, que l'on appellera centre angulaire de S1, on définit un angle écart angulaire maximal, $\theta_{1,errMax}$, comme le maximum de $\theta_{1,err}(P1,C)$ pour P1 parcourant l'ensemble de la surface S1.

L'importance de l'effet de confinement de la lumière en périphérie de la goutte, dont l'invention tire partie, dépend de l'épaisseur relative du "bol lumineux" qui vaut $\varepsilon = \dfrac{R - r}{R}$. Dans le cas d'une surface sphérique :

$$\varepsilon = 1 - \frac{n_{ext}}{n_{int}}.$$

De préférence, afin de rester dans le domaine de l'optique géométrique, l'épaisseur du bol lumineux doit être supérieure à la longueur d'onde $\lambda$ de la lumière. Autrement dit, de préférence, R - r $\geq \lambda$. Pour une longueur d'onde de $\lambda$ de 500 nm, et en supposant $n_{ext}$ = 1,33 (indice de réfraction de l'eau) et n = 1 (indice de réfraction de l'air), cette condition aboutit à R $\geq 2~\mu$m. Aussi, d'une façon générale, le diamètre d'une gouttelette G sera supérieur à 2 $\mu$m voire 4 $\mu$m.

Comme illustré sur la Figure 12B dans le cas d'une surface S1, pas tout-a-fait sphérique telle que caractérisée plus haut, un rayon limite pour la réflexion totale, Rlim, formera, dans le cas le plus défavorable de composition des angles, un angle $(\theta_c + \theta_{errMax})$ avec le segment [P1,C1]. L'épaisseur relative du bol lumineux dans cette configuration est

$\varepsilon^* = \dfrac{R^* - r^*}{R^*} = 1 - \sin(\theta_c + \theta_{errMax})$. En prenant des valeurs numériques correspondant à une goutte d'eau

dans l'air ($n_{int}/n_{ext}$ = 4/3, soit $\theta_c$= 48,6°), on trouve que $\theta_{errMax}$ = 4,5° correspond à une diminution relative la largeur de l'anneau de 20%. Si l'on tolère une diminution relative de la largeur de l'anneau de 50%, on peut tolérer des écarts angulaires sur S1 par rapport à une sphère allant jusqu'à 12,5°.

Un angle de contact (entre S1 et S2), noté $\theta_{cont}$, différent de 90° a pour conséquence que le rayon principal (RP) du faisceau des rayons qui sont confinés dans le bol lumineux, n'est plus perpendiculaire à S2 au moment de traverser cette surface (voir figure 12C). Le critère qui est pris pour définir la tolérance est que toute l'ouverture numérique du système d'observation (cône de demi-angle au sommet $\theta_{cont}$ hachuré sur la figure 12D) soit comprise dans l'angle d'ouverture du faisceau guidé, qui est limité par RL1 et RL2. En raisonnant entièrement dans le milieu d'indice $n_{int}$, l'angle d'ouverture du faisceau guidé est $\theta_{NAMax}$ = (90°-$\theta_c$), soit 41,4° pour $n_{int}/n_{ext}$ = 4/3 (couple eau - air). Pour que le triangle hachuré soit compris entre RL1 et LR2, il faut que |90°-$\theta_{cont}$| < $\theta_{NAMax}$ - $\theta_{0bs}$. Pour une collection de la lumière par un objectif de microscope d'ouverture numérique typique ON = 0,5, on a un angle d'ouverture $\theta_{obs}$ dans le milieu d'indice $n_{int}$ de 22° (ON = $n_{int} \sin \theta_{obs}$) Avec ce système de visualisation et pour $n_{int}/n_{ext}$ = 4/3, on trouve $\theta_{cont}$ compris entre 70 et 110°.

La gouttelette G peut être remplacée par un volume convexe et de forme généralement arrondie d'un solide ou d'un gel. Dans ces cas, la surface S2 peut comporter des facettes (en nombre suffisamment élevé, de manière indicative au moins 10 et de préférence 20 ou plus, pour qu'on puisse qualifier la surface de « généralement arrondie »).

Le milieu ambiant ne doit pas nécessairement être l'air, bien que ce choix soit avantageux en ce qu'il permet de maximiser la différence $n_{int}$-$n_{ext}$. En effet, l'effet de confinement de la lumière est d'autant plus efficace que la différence entre les indices de réfraction est importante ; de préférence on aura $n_{int} \geq 1,1 \cdot n_{ext}$.

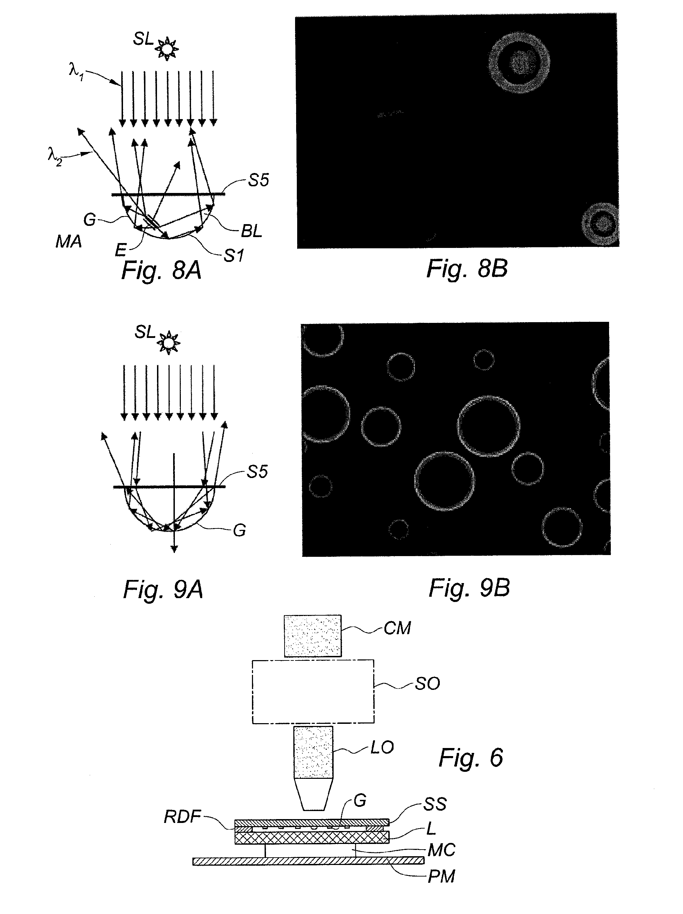

La figure 6 montre un montage expérimental pour la mise en oeuvre du procédé de l'invention.

Le substrat SS est une lamelle de verre de 170 $\mu$m d'épaisseur, silanisée de manière à être rendue hydrophobe. Une solution aqueuse contenant les objets ou substances à détecter (des microorganismes, des molécules fluorescentes en solution, etc.) est nébulisée sur cette lamelle, formant des gouttelettes de rayon compris entre quelques $\mu$m, par exemple 2 $\mu$m ou 4 $\mu$m, et 200$\mu$m, soit des volumes de 1pl à 10 nl environ. Du fait du traitement hydrophobe de la surface, ces gouttelettes présentent un angle de contact compris entre 92° et 96°(le cas hémisphérique, idéal, correspond à un angle de contact de 90°). Des simulations numériques ont permis de montrer que la force gravitationnelle ne déforme pas les gouttelettes, quelle que soit l'orientation de la lamelle.

Il faut noter que plusieurs techniques autres que la simple nébulisation peuvent être utilisées pour former les gouttelettes, en particulier si on souhaite qu'elles soient mono-dispersées. On peut citer à titre d'exemple le dépôt par « spotting », c'est-à-dire par dépôt d'une gouttelette (ou d'une pluralité de gouttelettes) en utilisant une micropipette, par exemple piézo-électrique, pouvant être pilotée par un automate, par dispersion ou par micro-structuration de zones hydrophiles/hydrophobes comme décrit dans le document US6391578. Le dépôt de gouttelettes de gel peut être réalisé selon la technique décrite dans le document WO82/02562, dans laquelle une suspension est fractionnée à l'état liquide, puis gélifiée.

On peut également envisager l'utilisation d'une ou plusieurs cuvettes de forme hémisphérique, destinées à être remplies d'un échantillon liquide ou gélifié. Dans ce cas, la cuvette a un indice de réfraction inférieur à celui de l'échantillon qu'elle est destinée à contenir. Par exemple, selon cette configuration, l'échantillon peut être un liquide ionique, dont l'indice est généralement supérieur à 1,4. Le matériau constituant la cuvette peut alors être un polymère d'indice inférieur à 1,4 sous la forme d'un bloc creusé de cuvettes sensiblement hémisphériques, ou bien d'un film fin thermoformé. Dans les deux cas, les cuvettes peuvent être fermées par assemblage d'un capot transparent formant la surface SS.

Après nébulisation, la lamelle SS est retournée sur un cadre constitué à l'aide d'un ruban adhésif double face RDF, par exemple du type « gene frame » (250 $\mu$m d'épaisseur), préalablement déposé sur une autre lamelle L. Le ruban adhésif forme alors des parois entre la lamelle SS et la lamelle S. On forme ainsi une chambre étanche fine, qui empêche l'évaporation des gouttelettes. Un moyen de chauffage MC est utilisé pour maintenir cette chambre à une température constante de 37°, et l'ensemble est monté sur une platine motorisée PM. Un microscope MS est utilisé pour observer les gouttelettes du côté de la lamelle supérieure SS. De préférence, l'éclairage et la collection de lumière produite par l'échantillon sont réalisés du même côté par rapport à la lamelle SS. Ainsi, l'éclairement de l'échantillon peut bénéficier à son tour de l'effet de confinement dans un « bol lumineux », mais cela n'est pas essentiel.

L'observation peut être réalisée à l'aide d'un système d'imagerie comprenant un objectif LO (par exemple : grossissement 10, ouverture numérique 0,5) et une caméra CM pour l'acquisition d'images. Sur la figure 6, la référence SO indique, de manière générale, les autres composantes du système d'imagerie (moyens d'éclairage, miroirs dichroïques, filtres...), variables en fonction de la technique d'observation utilisée. En effet, le principe de l'invention peut s'appliquer à plusieurs techniques d'observation différentes, comme cela sera décrit plus en détail ci-après à l'aide des figures 7A à 12. Bien entendu, cette liste de modes de réalisation n'est pas exhaustive. Dans un souci de simplicité, ces figures 7A à 12 ne montrent pas le système optique ou d'imagerie utilisé pour la mise en oeuvre du procédé.

La figure 7A illustre, de manière très schématique, l'application du procédé de l'invention à la détection d'un fluorophore dans des gouttelettes par microscopie à épifluorescence. La lumière d'excitation, à la longueur d'onde $\lambda_1$, est émise par une source SL (typiquement une lampe à mercure ou halogène pourvue d'un filtre) et injectée dans la gouttelette G par sa surface S2 d'adhérence au substrat SS. Cette lumière, partiellement confinée dans le « bol lumineux » BL induit la fluorescence du fluorophore, qui émet un rayonnement à la longueur d'onde $\lambda_2 > \lambda_1$. Comme on peut le voir sur la figure 7B, le rayonnement de fluorescence sort de la gouttelette, et du substrat, étant partiellement concentrée dans un anneau lumineux. On remarquera que les molécules du fluorophore qui se trouvent à l'extérieur du bol lumineux contribuent moins efficacement au signal détecté par la caméra CM, car elles sont exposées à un flux de lumière d'excitation moins intense et les photons qu'elles émettent ne sont pas confinés dans l'anneau lumineux. Le « bol lumineux » occupe une fraction du volume de la gouttelette d'autant plus grand que le rapport $n_{int}/n_{ext}$ est important.

Les images de la figure 7B ont été obtenues en excitant une solution du fluorophore 4MU dans le tampon MOPS (acide 3-(N-morpholino)propanesulfonic) à une longueur d'onde $\lambda_1$=360 nm. Le rayonnement de fluorescence a été isolé par un filtre centré à $\lambda_2$=450 nm

En variante, l'éclairage de la gouttelette pourrait se faire par la surface S1, ce qui conduirait à une excitation spatialement plus homogène dans la goutte, au détriment de l'effet de confinement du faisceau d'excitation dans un bol lumineux. On ne pourrait plus parler, dans ce cas, d'« épifluorescence ».

Le procédé d'observation des figures 7A et 7B peut être appliqué en particulier à l'étude de la cinétique de réactions chimiques ou biochimiques.

Les figures 8A et 8B se rapportent au cas de l'observation, toujours par microscopie à épifluorescence, d'un objet fluorescent de dimensions microscopiques E, tel qu'une bactérie, le cas échéant modifiée génétiquement. Si l'objet E est plus dense que le liquide formant la gouttelette, il tombe vers l'extrémité inférieure de cette dernière, et se positionne ainsi à l'intérieur du « bol lumineux ». Le confinement de la lumière d'excitation près de la surface S1 s'avère donc particulièrement avantageux. Sur la figure 8B on peut distinguer à la fois l'anneau dans lequel se concentrent les rayons lumineux émis par l'objet E et guidés par l'interface gouttelette/milieu ambiant, et l'image dudit objet formée par les rayons « directs », qui traversent le substrat sans réflexion préalable par la surface S1.

Dans le cas de cette figure 8B, l'objet E est une bactérie Escherichia coli modifiée génétiquement dans le milieu de croissance contenant du MUG (4MU glucuronate). L'observation est réalisée avec $\lambda_1$=557 nm (longueur d'onde d'excitation) et $\lambda_2$=579 nm (longueur d'onde de fluorescence).

Un procédé d'observation selon les figures 8A et 8B peut être appliqué à la numération de bactéries inoculées dans une solution qui est nébulisée comme décrit précédemment pour former un ensemble de gouttelettes sur une lamelle de microscope, les bactéries étant réparties de façon aléatoire dans les gouttelettes. La solution peut comprendre un fluorophore de référence, par exemple du FITC (fluorescéine Isothiocyanate) qui permet de repérer les gouttes, estimer leur volume et normaliser le signal de fluorescence des bactéries de manière à s'affranchir des dérives instrumentales.

Ce procédé a été testé en utilisant une souche d'Escherichia coli ATCC 11775. Il s'agit d'une souche β-glucuronidase-positive. L'activité enzymatique glucuronidase permet la dégradation du substrat fluorogène MUG, ce qui entraîne la libération de fluorophore 4MU. La solution aqueuse dans laquelle ces bactéries sont inoculées est un tampon de contenant du MOPS (acide 3-(N-morpholino)propanesulfonic), le substrat fluorogène MUG ainsi que de la FITC en tant que fluorophore de référence. La composition de ce milieu est :

- MOPS (4-morpholinepropanesulfonicacidsodiumsalt) 150mM;
- magnesiumsulfate2,6mM;
- sodiumglucuronate 854 $\mu$M;
- methyl β-d-glucuronide 870 $\mu$M;
- MUG(4-methylumbelliferyl-β-d-glucuronide) 113 $\mu$M;
- fluoresceine 4,15 $\mu$M.

L'observation des gouttelettes est réalisée en utilisant un microscope AxioImager de Zeiss avec un objectif corrigé de l'épaisseur de la lamelle x10 ON 0,5. Le MUG fait office de traceur de chaque bactérie, du fait de la production de fluorophore 4MU dans les gouttelettes contenant au moins une bactérie. Le fluorophore FITC fait office de traceur de chaque goutte, car chaque goutte en émet, indépendamment de la présence d'une bactérie.

Le fluorophore 4MU étant produit par le métabolisme des bactéries, son signal de fluorescence augmente dans le temps. Par ailleurs, le tampon contient une concentration donnée de fluorophore FITC, qui reste constante tout au long de la mesure : le signal de fluorescence du FITC est donc constant et peut être utilisé en tant que signal de référence.

Durant l'observation :

- on excite alternativement l'échantillon aux longueurs d'onde d'excitation du FITC et du fluorophore 4MU (à compléter)
- on enregistre, alternativement, les images correspondant aux longueurs d'onde d'émission du FITC et du fluorophore 4MU
- Sur chaque image d'émission du FITC, on localise les gouttes, qui sont toutes fluorescentes, chacune produisant un anneau sur l'image. Chaque anneau définit une zone spatiale d'intérêt correspondant à chaque goutte. D'autre part, on peut mesurer le rayon extérieur, Rext, de chaque anneau d'une même image, ce rayon étant corrélé au volume de chaque goutte selon l'expression suivante :

$$V\left(R_{ext}, \theta_{cont}\right) = \frac{\pi}{3}\left(\frac{R_{ext}}{\sin(\theta_{cont})}\right)^3 \left(2 - 3\cos(\theta_{cont}) + \cos^3(\theta_{cont})\right)$$

Avec:

- $\theta_{cont}$ = angle de contact de la goutte sur la lamelle SS
- $R_{ext}$ = diamètre extérieur de l'anneau lumineux

Sur chaque image d'émission du fluorophore 4MU, on utilise les zones spatiales d'intérêt préalablement définies. On détermine l'intensité moyenne du signal des pixels constituant chaque zone spatiale d'intérêt, cette intensité moyenne étant l'intensité moyenne du signal produit par chaque goutte. De façon optionnelle, on peut normaliser l'intensité moyenne du signal produit par chaque goutte, à la longueur d'onde d'émission du fluorophore 4Mu, par l'intensité moyenne du signal correspondant à la même goutte, à la longueur d'onde d'émission du FITC. Par seuillage de l'intensité, ou de l'intensité normalisée, on détermine les gouttes contenant au moins une bactérie, le seuillage permettant de conclure sur la présence ou l'absence d'au moins une bactérie dans la goutte.

En appliquant une loi Poissonnienne, on peut déterminer la concentration de bactéries la plus probable (CPP) dans la solution initiale, c'est-à-dire la solution dispersée sous forme de gouttes.

On peut démontrer que

$$\sum_{j\,/\,Goutte\,occupée} \frac{Vj}{1 - e^{-CPP.Vi}} = \sum_{i\,/\,Goutte\,observées} Vi$$

- Vj étant le volume élémentaire de chaque goutte j occupée par au moins une bactérie.
- Vi étant le volume élémentaire de chaque goutte i observée par fluorescence du FITC
- CPP étant la concentration la plus probable de bactéries dans la solution initiale. La dimension de cette concentration est l'inverse d'un volume.

Dans un essai, une valeur de 5,7 $10^5$ bactéries/ml a été déterminée en moins de 4 heures. La concentration exacte en bactéries n'étant pas connue, une numération sur boîte CPS3 (fournisseur bioMérieux) après plus de 24 heures de culture a été effectuée à titre de référence. Cette méthode de comptage donne un résultat de 6,5 $10^5$ bactéries/ml. Ce résultat est en bon accord avec la méthode référence puisque l'écart observé est en effet inférieur au facteur 2 généralement toléré

Les figures 9A et 9B montrent des images de gouttelettes observées par microscopie en réflexion (éclairage et détection à travers le substrat SS à une même longueur d'onde $\lambda$). Cela peut être utilisé, par exemple, pour vérifier le fonctionnement de dispositifs microfluidiques, pour étudier la mouillabilité du substrat ou l'indice de réfraction du liquide formant la gouttelette.

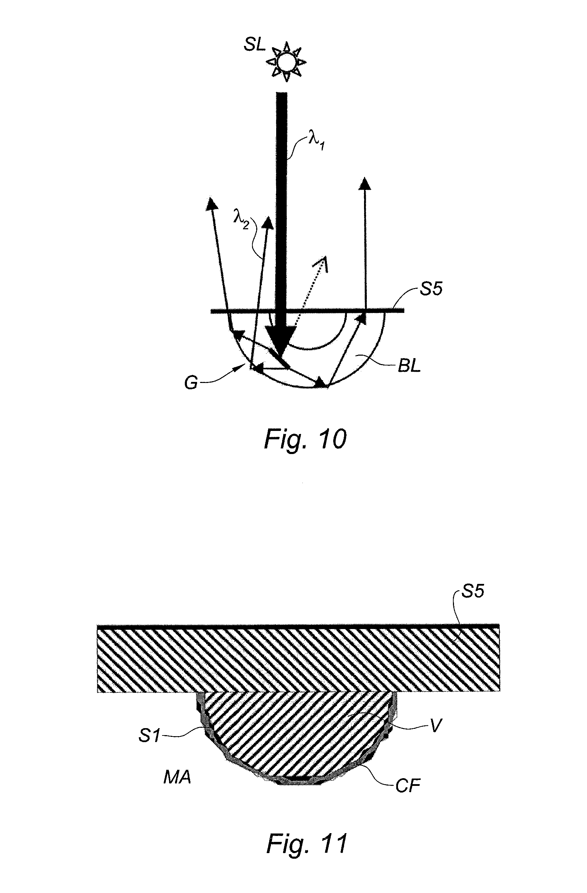

Le guidage de la lumière qui se produit à l'interface entre la gouttelette et le milieu ambiant peut être exploité pour permettre une sensibilité accrue lors de l'observation par des méthodes de spectroscopie optique (par exemple la fluorescence intrinsèque et la diffusion inélastique Raman) d'objets ou d'analytes biologiques. Pour les analytes on se trouve dans une configuration identique à la fluorescence (figure 7A). Pour des objets uniques E on peut se trouver aussi bien dans la configuration de la fluorescence (figure 8A) que dans une nouvelle configuration où la lumière d'excitation est limitée à un pinceau impactant l'objet (voir figure 10).

Toutes les méthodes d'observations décrites jusqu'ici impliquent un éclairage de l'échantillon, que ce soit à travers la surface convexe S1 ou - ce qui est généralement préférable - à travers la surface plane S2. Cependant, un tel éclairage n'est pas nécessaire lorsque l'échantillon est luminescent, ce qui est le cas de certains microorganismes ou de certaines solutions dans lesquelles se produisent des réactions chimiques provoquant l'émission de lumière (réactions de chimioluminescence catalysées par des enzymes telles que la phosphatase alcaline ou la peroxydase de raifort (« horseradish peroxydase »), ou des micro-organisme modifiés comportant une activité luciférase, tel que décrits par exemple dans l'article de M. L. Eldridge et al « Saccharomyces cerevisiae BLYAS, a New Bioluminescent Bioreporter for Detection of Androgenic Compounds » APPLIED AND ENVIRONMENTAL MICROBIOLOGY, 73(19), p. 6012-6018 (2007). Des simulations ont été entreprises, afin de comparer le signal lumineux détecté par un détecteur, placé à distance d'une goutte, collectant le signal lumineux produit à l'intérieur de cette dernière de manière uniforme et isotrope, dans les deux configurations suivantes :

- une goutte hémisphérique telle que représentée sur la figure 3A, la goutte étant formée d'une solution aqueuse comportant une concentration donnée de molécule chimiluminescence,
- une goutte sphérique, de même volume, et de même composition, baignant dans un bain d'huile, selon la configuration représentée sur la figure 4A.

D'après ces calculs, le signal collecté est 2,5 à 2,8 fois plus important lorsque la goutte est hémisphérique, pour des ouvertures numériques du détecteur variant entre 0,1 et 0,6. Ainsi, la configuration hémisphérique d'obtenir un signal plus intense.

La figure 11 se rapport à un cas particulier, où une protubérance solide V de forme hémisphérique (une « demi-bille ») et présentant une couche fonctionnalisée CF déposée sur sa surface S1 est utilisée comme capteur chimique. Des espèces chimiques formant ou contenues dans le milieu ambiant MA (liquide ou gazeux) réagissent avec la couche fonctionnalisée CF pour produire un signal optique détectable, tel qu'un signal de fluorescence ou de luminescence, ou pour modifier l'indice de réfraction de cette dernière, ce qui à son tour affecte le rayon interne de l'anneau lumineux (observation dans la configuration de la figure 9A). En variante, ce peut être le matériau même constituant la protubérance V qui réagit avec le milieu ambiant. Dans ce cas, la protubérance peut être remplacée par une gouttelette, ou être constituée par un gel.

## Revendications

1. Procédé d'observation d'un échantillon comprenant les étapes consistant à :

   a) procurer un volume (G, V) d'un milieu sensiblement transparent ayant un premier indice de réfraction ($n_{int}$) et présentant une première surface (S1) convexe, arrondie ou à facettes, et une deuxième surface (S2) sensiblement plane, ladite première surface séparant ledit volume d'un milieu ambiant (MA) présentant un deuxième indice de réfraction ($n_{ext}$) inférieur audit premier indice, l'échantillon à observer étant contenu dans ledit volume ou déposé sur ladite première surface ;
   b) observer ledit échantillon à travers ladite deuxième surface sensiblement plane ; et
   c) déterminer la présence d'une substance ou d'un objet dans l'échantillon par la détection, lors de ladite étape b), d'un anneau lumineux en correspondance dudit volume.

**2.** Procédé selon la revendication 1, dans lequel ledit volume présente une forme sensiblement hémisphérique.

**3.** Procédé selon l'une des revendications précédentes, dans lequel ladite étape c) comprend la détermination de la présence d'un micro-organisme dans ledit échantillon.

**4.** Procédé selon l'une des revendications précédentes, dans lequel ledit volume est une gouttelette adhérée à un substrat transparent (SS) formant ladite deuxième surface sensiblement plane.

**5.** Procédé selon la revendication 4 dans lequel :

- ladite étape a) comporte la réalisation d'une pluralité de gouttelettes formant desdits volumes dont au moins certains contiennent au moins un objet ou une substance à détecter ; et
- ladite étape b) comporte l'observation simultanée d'une pluralité desdites gouttelettes.

**6.** Procédé selon l'une des revendications 1 ou 2, dans lequel ledit volume est un volume solide et l'échantillon est constitué par une couche fonctionnalisée (CF), sensible à un analyte pouvant être contenu dans ledit milieu ambiant, déposée sur sa première surface.

**7.** Procédé selon l'une des revendications 1 ou 2, dans lequel ledit volume est en un matériau sensible à un analyte pouvant être contenu dans ledit milieu ambiant.

**8.** Procédé selon l'une des revendications précédentes, dans lequel ledit milieu ambiant est l'air.

**9.** Procédé selon l'une des revendications précédentes, dans lequel ladite étape b) comporte une opération d'éclairage dudit volume.

**10.** Procédé selon la revendication 9, dans lequel ledit éclairage est effectué du côté de ladite deuxième surface sensiblement plane.

**11.** Procédé selon l'une des revendications 9 ou 10, dans lequel ladite étape b) comporte la détection d'un signal de fluorescence.

**12.** Procédé selon la revendication 11, dans lequel ladite étape b) est mise en oeuvre par microscopie à épifluorescence.

**13.** Procédé selon l'une des revendications 9 ou 10, dans lequel ladite étape b) comporte la détection d'un signal de diffusion Raman.

**14.** Procédé selon l'une des revendications 9 ou 10, dans lequel ladite étape b) est mise en oeuvre par microscopie à réflexion ou à transmission.

**Patentansprüche**

**1.** Verfahren zur Beobachtung einer Probe, umfassend die Schritte bestehend aus:

a) Beschaffen eines Volumens (G, V) eines im Wesentlichen transparenten Mediums, welches einen ersten Brechungsindex ($n_{int}$) aufweist und eine konvexe, abgerundete oder facettierte erste Oberfläche (S1) und eine im Wesentlichen ebene zweite Oberfläche (S2) aufweist, wobei die erste Oberfläche das Volumen von einem umgebenden Medium (MA) trennt, welches einen zweiten Brechungsindex ($n_{ext}$) kleiner als der erste Brechungsindex aufweist, wobei die zu beobachtende Probe in dem Volumen enthalten oder auf der ersten Oberfläche angeordnet ist;
b) Beobachten der Probe durch die im Wesentlichen ebene zweite Oberfläche; und
c) Bestimmen des Vorhandenseins einer Substanz oder eines Objekts in der Probe durch die Erfassung, bei dem Schritt b), eines dem Volumen entsprechenden Lichtrings.

**2.** Verfahren nach Anspruch 1, wobei das Volumen eine im Wesentlichen halbkugelförmige Form aufweist.

**3.** Verfahren nach einem der vorhergehenden Ansprüche, wobei der Schritt c) die Bestimmung des Vorhandenseins eines Mikroorganismus in der Probe umfasst.

**4.** Verfahren nach einem der vorhergehenden Ansprüche, wobei das Volumen ein Tröpfchen ist, welches an einem transpa-renten Substrat (SS) anhaftet, welches die im Wesentlichen ebene zweite Oberfläche bildet.

**5.** Verfahren nach Anspruch 4, wobei:

- der Schritt a) die Realisierung einer Vielzahl von Tröpfchen umfasst, welche genannte Volumina bilden, von welchen wenigstens einige wenigstens ein zu erfassendes Objekt oder eine zu erfassende Substanz enthalten; und
- der Schritt b) die gleichzeitige Beobachtung einer Vielzahl der Tröpfchen umfasst.

**6.** Verfahren nach einem der Ansprüche 1 oder 2, wobei das Volumen ein festes Volumen ist und die Probe durch eine funktionalisierte Schicht (CF) gebildet ist, welche sensitiv auf ein Analyt, das in dem umgebenden Medium enthalten sein kann, welches auf seiner ersten Oberfläche angeordnet ist.

**7.** Verfahren nach einem der Ansprüche 1 oder 2, wobei das Volumen ein Material ist, welches sensitiv auf ein Analyt ist, welches in dem umgebenden Medium enthalten sein kann.

**8.** Verfahren nach einem der vorhergehenden Ansprüche, wobei das umgebende Medium Luft ist.

**9.** Verfahren nach einem der vorhergehenden Ansprüche, wobei der Schritt b) einen Vorgang einer Beleuchtung des Volumens umfasst.

**10.** Verfahren nach Anspruch 9, wobei die Beleuchtung von der Seite der im Wesentlichen ebenen zweiten Oberfläche bewerkstelligt wird.

**11.** Verfahren nach einem der Ansprüche 9 oder 10, wobei der Schritt b) die Erfassung eines Fluoreszenzsignals umfasst.

**12.** Verfahren nach Anspruch 11, wobei der Schritt b) durch Epifluoreszenzmikroskopie umgesetzt wird.

**13.** Verfahren nach einem der Ansprüche 9 oder 10, wobei der Schritt b) die Erfassung eines Raman-Streuungssignals umfasst.

**14.** Verfahren nach einem der Ansprüche 9 oder 10, wobei der Schritt b) durch Reflexions- oder Transmissionsmikroskopie umgesetzt wird.

**Claims**

**1.** A method for observing a sample, comprising the steps consisting of:

a) providing a volume (G, V) of a substantially transparent medium having a first refractive index ($n_{int}$) and having a first convex, rounded or facetted surface (S1), and a second substantially planar surface (S2), said first surface separating said volume from an ambient medium (MA) having a second refractive index ($n_{ext}$) that is lower than said first index, the sample to be observed being constituted by said volume or disposed within it or deposited on said first surface;
b) observing said sample through said second substantially planar surface; and
c) determining the presence of a substance or an object in the sample by detection during said step b) of a luminous ring matched to said volume.

**2.** The method as claimed in claim 1, in which said volume has a substantially hemispherical shape.

**3.** The method as claimed in one of the preceding claims, in which said step c) comprises determining the presence of a microorganism in said sample.

**4.** The method as claimed in one of the preceding claims, in which said volume is a droplet adhered to a transparent substrate (SS) forming said second substantially planar surface.

5. The method as claimed in claim 4, in which:

   • said step a) comprises producing a plurality of droplets forming said volumes at least some of which contain at least one object or a substance to be detected; and
   • said step b) comprises simultaneously observing a plurality of said droplets.

6. The method as claimed in claim 1 or claim 2, in which said volume is a solid volume and the sample is constituted by a functionalized layer (CF) that is sensitive to an analyte that might be contained in said ambient medium deposited on its first surface.

7. The method as claimed in claim 1 or claim 2, in which said volume is formed from a material that is sensitive to an analyte that might be contained in said ambient medium.

8. The method as claimed in one of the preceding claims, in which said ambient medium is air.

9. The method as claimed in one of the preceding claims, in which said step b) comprises an operation for illuminating said volume.

10. The method as claimed in claim 9, in which said illumination is carried out from the side of said substantially planar second surface.

11. The method as claimed in claim 9 or claim 10, in which said step b) comprises detecting a fluorescence signal.

12. The method as claimed in claim 11, in which said step b) is carried out by epifluorescence microscopy.

13. The method as claimed in claim 9 or claim 10, in which said step b) comprises detecting a Raman diffusion signal.

14. The method as claimed in claim 9 or claim 10, in which said step b) is carried out using reflection or transmission microscopy.

*Fig. 1*

*Fig. 2A*

*Fig. 2B*

12

Fig. 3

Fig. 4B

Fig. 4A

**Fig. 5**

**Fig. 7A**

**Fig. 7B**

Fig. 8A

Fig. 8B

Fig. 9A

Fig. 9B

Fig. 6

*Fig. 10*

*Fig. 11*

*Fig. 12A*

*Fig. 12B*

*Fig. 12C*

*Fig. 12D*

## RÉFÉRENCES CITÉES DANS LA DESCRIPTION

**Documents brevets cités dans la description**

- US 20050221339 A
- US 6391578 B
- WO 8202562 A

**Littérature non-brevet citée dans la description**

- **A. B. THEBERGE et al.** Microdroplets in Microfluidics : An Evolving Platform for Discoveries in Chemistry and Biology. *Angew. Chem. Int. Ed.,* 2010, vol. 49, 5846-5868
- **B. ROTMAN.** Measurement of activity of single molecules of β-D-Galactosidase. *Proceedings of the Natl. Acad. Sci. USA,* 1961, vol. 47, 1981-1991
- **M. L. ELDRIDGE et al.** Saccharomyces cerevisiae BLYAS, a New Bioluminescent Bioreporter for Detection of Androgenic Compounds. *APPLIED AND ENVIRONMENTAL MICROBIOLOGY,* 2007, vol. 73 (19), 6012-6018